(19) Europäisches Patentamt
European Patent Office
Office européen des brevets

(11) **EP 2 136 918 B1**

(12) **EUROPÄISCHE PATENTSCHRIFT**

(45) Veröffentlichungstag und Bekanntmachung des Hinweises auf die Patenterteilung:
**11.12.2019 Patentblatt 2019/50**

(21) Anmeldenummer: **08735610.1**

(22) Anmeldetag: **31.03.2008**

(51) Int Cl.:
*B01J 35/02* (2006.01)     *B01J 35/08* (2006.01)
*B01J 8/06* (2006.01)      *B01J 23/887* (2006.01)
*B01J 27/198* (2006.01)    *C07C 51/25* (2006.01)
*B01J 23/28* (2006.01)     *B01J 23/50* (2006.01)
*B01J 23/888* (2006.01)    *C07C 45/35* (2006.01)
*C07C 51/265* (2006.01)    *B01J 35/00* (2006.01)
*B01J 19/30* (2006.01)     *B01J 23/00* (2006.01)
*B01J 8/00* (2006.01)      *B01J 37/00* (2006.01)

(86) Internationale Anmeldenummer:
**PCT/EP2008/053816**

(87) Internationale Veröffentlichungsnummer:
**WO 2008/122537 (16.10.2008 Gazette 2008/42)**

(54) **VERFAHREN ZUR BESCHICKUNG EINES LÄNGSABSCHNITTS EINES KONTAKTROHRES**

METHOD FOR FEEDING A LONGITUDINAL SECTION OF A CONTACT PIPE

PROCÉDÉ POUR CHARGER UNE PARTIE LONGITUDINALE D'UN TUBE DE CONTACT

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **10.04.2007 DE 102007017080**
**10.04.2007 US 910908 P**

(43) Veröffentlichungstag der Anmeldung:
**30.12.2009 Patentblatt 2009/53**

(73) Patentinhaber: **BASF SE**
**67056 Ludwigshafen am Rhein (DE)**

(72) Erfinder:
• **DIETERLE, Martin**
**Jersey City, NJ 07302 (US)**
• **MÜLLER-ENGEL, Klaus Joachim**
**76297 Stutensee (DE)**

(74) Vertreter: **BASF IP Association**
**BASF SE**
**G-FLP-C006**
**67056 Ludwigshafen (DE)**

(56) Entgegenhaltungen:
EP-A- 1 270 065          GB-A- 1 550 036
US-A- 4 366 093          US-A1- 2004 225 138
US-A1- 2004 260 103      US-A1- 2006 205 978

EP 2 136 918 B1

**Beschreibung**

Vorliegende Erfindung betrifft ein Verfahren zur Beschickung eines Längsabschnitts eines Kontaktrohres gemäß Anspruch 1, ein Verfahren zur Beschickung eines Kontaktrohres gemäß Anspruch 4, einen Rohrbündelreaktor gemäß Anspruch 5, und ein Verfahren zur heterogen katalysierten partiellen Gasphasenoxidation einer organischen Verbindung gemäß Anspruch 6.

US 2004/225138 A1 offenbart Reaktorsysteme zur Herstellung von Ethylenoxid. Die bevorzugte katalytische Komponente ist Silber.

EP 1 270 065 A offenbart ein Verfahren zur Herstellung von (Meth) Acrylsäure und / oder (Meth) Acrolein. In einem Beispiel enthält der verwendete Katalysator Molybdän und Vanadium.

GB 1 550 036 A betrifft ein Verfahren zur Herstellung von Phthalsäureanhydrid unter Verwendung eines Vanadiumpentoxid enthaltenden Festbettkatalysators.

US 2004/260103 A1 betrifft silberhaltige Katalysatorzusammensetzungen, die besonders zur Verwendung bei der Herstellung von Ethylenoxid geeignet sind. Es ist allgemein bekannt, heterogen katalysierte partielle Gasphasenoxidationen am in den meist vertikal angeordneten Rohren (den sogenannten Kontaktrohren) von Rohrbündelreaktoren (Reaktoren, die ein in einem Reaktionsbehälter enthaltenes Bündel von Kontaktrohren aufweisen) befindlichen Katalysatorfestbett durchzuführen.

Unter einer vollständigen Oxidation einer organischen Verbindung mit molekularem Sauerstoff wird in dieser Schrift verstanden, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen exothermen Umsetzungen einer organischen Verbindung unter reaktiver Einwirkung von molekularem Sauerstoff werden hier als Partialoxidationen einer organischen Verbindung zusammengefasst.

Im besonderen sollen in dieser Schrift unter Partialoxidationen solche exothermen Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält, als vor Durchführung der Partialoxidation.

Die für vorgenannte heterogen katalysierte partielle Gasphasenoxidationen benötigten Rohrbündelreaktoren sind ebenso bekannt (vgl. z. B. DE-A 44 31 949, EP-A 700 714).

Es wird bei diesen Reaktionen das Reaktionsgasgemisch durch das in den Kontaktrohren des Rohrbündelreaktors befindliche Katalysatorfestbett geführt und während der Verweilzeit der Reaktanden an der Katalysatoroberfläche setzen sich die Reaktanden um.

Die Reaktionstemperatur in den Kontaktrohren wird u. a. dadurch kontrolliert, dass man um die in einem Behälter untergebrachten Kontaktrohre des Rohrbündels einen fluiden Wärmeträger (ein Wärmeaustauschmittel) führt, um aus dem Reaktionssystem Energie abzuführen. Dabei können Wärmeträger und Reaktionsgasgemisch über den Rohrbündelreaktor sowohl im Gleichstrom als auch im Gegenstrom geführt werden.

Neben der Möglichkeit, das Wärmeaustauschmittel dabei in einfacher Weise im wesentlichen unmittelbar längs zu den Kontaktrohren zu führen, kann diese Längsführung auch lediglich über den gesamten Reaktionsbehälter verwirklicht und dieser Längsströmung innerhalb des Reaktionsbehälters durch eine längs der Kontaktrohre aufeinanderfolgende Anordnung von Durchtrittsquerschnitte frei lassenden Umlenkscheiben eine Querströmung so überlagert werden, dass im Längsschnitt durch das Rohrbündel ein mäanderförmiger Strömungsverlauf des Wärmeaustauschmittels resultiert (vgl. z. B. DE-A 44 31 949, EP-A 700 714, DE-PS 28 30 765, DE-A 22 01 528, DE-A 22 31 557 sowie DE-A 23 10 517).

Bei Bedarf können um die Kontaktrohre längs voneinander verschiedener Rohrabschnitte voneinander räumlich im wesentlichen getrennte Wärmeträger geführt werden.

Der Rohrabschnitt, über den sich der jeweilige Wärmeträger erstreckt, repräsentiert dabei üblicherweise eine eigene Reaktionszone. Eine bevorzugt eingesetzte Variante solcher Mehrzonenrohrbündelreaktoren ist der Zweizonenrohrbündelreaktor, wie ihn z. B. die Schriften DE-C 28 30 765, DE-C 25 13 405, US 3,147,084, DE-A 22 01 528, EP-A 383224 und DE-A 29 03 582 beschreiben.

Als Wärmeaustauschmittel eignen sich z. B. Schmelzen von Salzen wie Kaliumnitrat, Kaliumnitrit, Natriumnitrit und/oder Natriumnitrat, niedrig schmelzende Metalle wie Natrium, Quecksilber sowie Legierungen verschiedener Metalle, ionische Flüssigkeiten (in denen wenigstens eines der entgegengesetzt geladenen Ionen wenigstens ein Kohlenstoffatom enthält), aber auch konventionelle Flüssigkeiten wie z.B. Wasser oder hochsiedende organische Lösungsmittel (z. B. Mischungen aus Diphyl® und Dimethylphthalat).

Üblicherweise sind die Kontaktrohre aus ferritischem Stahl oder aus Edelstahl gefertigt und weisen häufig eine Wanddicke von einigen mm, z. B. 1 bis 3 mm auf. Ihr Innendurchmesser beträgt meist einige cm, z. B. 10 bis 50 mm, häufig 20 bis 30 mm. Die Rohrlänge erstreckt sich im Normalfall auf wenige Meter (typisch ist eine Kontaktrohrlänge im Bereich von 1 bis 8 m, häufig 2 bis 6 m, vielfach 2 bis 4 m). Anwendungstechnisch zweckmäßig beläuft sich die im Behälter untergebrachte Anzahl an Kontaktrohren (Arbeitsrohren) auf wenigstens 1000, häufig wenigstens 3000 oder 5000 und vielfach auf wenigstens 10000. Häufig beträgt die Anzahl der im Reaktionsbehälter untergebrachten Kontaktrohre 15000 bis 30000 oder 40000 bzw. 50000. Rohrbündelreaktoren mit einer oberhalb von 50000 liegenden Anzahl von Kontaktrohren bilden eher die Ausnahme. Innerhalb des Behälters sind die Kontaktrohre im Normalfall im wesentlichen homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 25 bis 55 mm, häufig 35 bis 45 mm beträgt (vgl. z.B. EP-A 468 290).

Im Normalfall sind jeweils wenigstens Teilmengen der Kontaktrohre (Arbeitsrohre) eines Rohrbündelreaktors, anwendungstechnisch zweckmäßig ihre Gesamtmenge, im Rahmen der Fertigungsmöglichkeiten einheitlich gefertigt. D.h., ihr Innendurchmesser, ihre Wanddicke und ihre Rohrlänge sind innerhalb enger Toleranzen identisch (vgl. WO 03/059857).

Das vorgenannte Anforderungsprofil trifft häufig auch auf die Befüllung solcher einheitlich gefertigten Kontaktrohre mit Katalysatorformkörpern zu (vgl. z. B. WO 03/057653), um einen optimalen und möglichst störungsfreien Betrieb des Rohrbündelreaktors zu gewährleisten. Insbesondere für eine optimale Ausbeute und Selektivität der im Rohrbündelreaktor durchgeführten Reaktionen ist es wesentlich, dass, vorzugsweise alle, Arbeitsrohre des Reaktors möglichst einheitlich mit dem Katalysatorfestbett befüllt, d.h., beschickt sind.

Arbeitsrohre werden üblicherweise von Thermorohren unterschieden, wie sie z. B. die EP-A 873 783 beschreibt. Während die Arbeitsrohre diejenigen Kontaktrohre sind, in denen die durchzuführende chemische Reaktion im eigentlichen Sinne durchgeführt wird, dienen Thermorohre in erster Linie dem Zweck, die Reaktionstemperatur in den Kontaktrohren zu verfolgen und zu steuern. Zu diesem Zweck enthalten die Thermorohre normalerweise zusätzlich zum Katalysatorfestbett eine lediglich mit einem Temperaturmessfühler beschickte, im Thermorohr längs desselben zentriert geführte Thermohülse. Im Regelfall ist die Anzahl der Thermorohre in einem Rohrbündelreaktor sehr viel kleiner als die Anzahl der Arbeitsrohre. Normalerweise beträgt die Anzahl der Thermorohre $\leq$ 20.

Als heterogen katalysierte partielle Oxidationen organischer Verbindungen beispielhaft genannt seien die Umsetzung von Propen zu Acrolein und/oder Acrylsäure (vgl. z. B. die DE-A 23 51 151), die Umsetzung von tert.-Butanol, iso-Buten, iso-Butan, iso-Butyraldehyd oder dem Methylether des tert. Butanols zu Methacrolein und/oder Methacrylsäure (vgl. z. B. DE-A 25 26 238, EP-A 92 097, EP-A 58 927, DE-A 41 32 263, DE-A 41 32 684 und DE-A 40 22 212), die Umsetzung von Acrolein zu Acrylsäure, die Umsetzung von Methacrolein zu Methacrylsäure (vgl. z. B. DE-A 25 26 238), die Umsetzung von o-Xylol oder Naphtalin zu Phtalsäureanhydrid (vgl. z. B. EP-A 522 871) sowie die Umsetzung von Butadien zu Maleinsäureanhydrid (vgl. z. B. DE-A 21 06 796  und DE-A 16 24 921), die Umsetzung von n-Butan zu Maleinsäureanhydrid (vgl. z. B. GB-A 1 464 198 und GB-A 1 291 354), die Umsetzung von Indanen zu z.B. Anthrachinon (vgl. z. B. DE-A 20 25 430), die Umsetzung von Ethylen zu Ethylenoxid oder von Propylen zur Propylenoxid (vgl. z. B. DE-AS 12 54 137, DE-A 21 59 346, EP-A 372 972, WO 89/0710, DE-A 43 11 608), die Umsetzung von Propylen und/oder Acrolein zu Acrylnitril (vgl. z. B. DE-A 23 51 151), die Umsetzung von iso-Buten und/oder Methacrolein zu Methacrylnitril (d. h., der Begriff der partiellen Oxidation soll in dieser Schrift auch die partielle Ammoxidation, d.h., eine partielle Oxidation im Beisein von Ammoniak, umfassen), die oxidative Dehydrierung von Kohlenwasserstoffen (vgl. z. B. DE-A 23 51 151), die Umsetzung von Propan zu Acrylnitril oder zu Acrolein und/oder Acrylsäure (vgl. z. B. DE-A 101 31 297, EP-A 1 09 0684, EP-A 608 838, DE-A 100 46 672, EP-A 529 853, WO 01/96270 und DE-A 100 28 582) etc..

Bei den Aktivmassen der zur Durchführung von exothermen heterogen katalysierten partiellen Gasphasenoxidation organischer Verbindungen zu verwendenden Katalysatoren handelt es sich in der Regel um wenigstens ein Multielementoxid, das

a) die Elemente Mo, Fe und Bi, oder
b) die Elemente Mo und V, oder
c) das Element V sowie zusätzlich P und/oder Sb

enthält, oder um Systeme, die elementares Silber auf einem oxidischen Träger enthalten.

Diese Aktivmassen werden zu Formkörpern unterschiedlichster Geometrie geformt (zu sogenannten geometrischen Katalysatorformkörpern) verwendet, um in den Rohren der Rohrbündelreaktoren das Katalysatorfestbett zu gestalten (die Kontaktrohre mit dem Katalysatorfestbett zu beschicken). Beispielsweise kommen als solche geometrischen Formkörper Kugeln, Tabletten, Stränge, Ringe, Spiralen, Pyramiden, Zylinder, Prismen, Quader, Würfel, etc. in Betracht.

Dabei kann der geometrische Formkörper im einfachsten Fall nur aus katalytisch aktiver Masse, die gegebenenfalls mit inertem Material verdünnt sein kann, bestehen. Solche geometrischen Katalysatorformkörper werden üblicherweise als Vollkatalysatoren bezeichnet.

Im Fall von Vollkatalysatoren kann die Formgebung z. B. dadurch erfolgen, dass man katalytisch aktive Pulvermasse (z. B. eine pulverförmige Multielementoxidaktivmasse) zur gewünschten Katalysatorgeometrie verdichtet (z. B. durch Tablettieren, Sintern, Extrudieren oder Strangpressen). Dabei können Formungshilfsmittel zugesetzt werden.

Alternativ kann man eine pulverförmige Vorläufermasse zur gewünschten Katalysatorgeometrie verdichten und den resultierenden geometrischen Formkörper durch thermisches Behandeln (gegebenenfalls in molekularen Sauerstoff enthaltender Atmosphäre) in den katalytisch aktiven Multielementoxidformkörper überführen (vgl. z. B. US 2005/0263926).

Selbstverständlich kann die Formgebung auch so erfolgen, dass man einen geometrischen Formkörper aus katalytisch nicht aktivem Material (aus Inertmaterial) mit Aktivmasse beschichtet (nachstehend auch als "Trägerformkörper" oder kurz als "Trägerkörper" bezeichnet). Alternativ kann auch mit Vorläufermasse beschichtet werden und die Überführung in den aktiven Katalysator durch nachträgliche thermische Behandlung erfolgen (gegebenenfalls in molekularen Sauerstoff enthaltender Atmosphäre). Das Beschichten kann in einfachster Weise z. B. dadurch erfolgen, dass man die Oberfläche eines inerten Trägerkörpers mittels eines flüssigen Bindemittels befeuchtet und nachfolgend pulverförmige Aktivmasse oder pulverförmige Vorläufermasse an der befeuchteten Oberfläche anheftet. Die auf diese Wiese erhältlichen Katalysatoren werden als Schalenkatalysatoren bezeichnet.

Geeignete inerte Trägerkörper sind für viele heterogen katalysierte partielle Gasphasenoxidationen poröse oder unporöse Aluminiumoxide, Siliciumoxid, Thoriumdioxid, Zirkonoxid, Siliciumcarbid oder Silikate wie Magnesium- oder Aluminiumsilicat (z. B. Steatit des Typs C220 der Fa. CeramTec), aber auch Metalle wie z. B. Edelstahl oder Aluminium (vgl. z. B. US 2006/0205978).

Anstelle den inerten (inert heißt hier in der Regel, dass wenn das Reaktionsgasgemisch untern den Reaktionsbedingungen durch ein nur mit inerten Trägerkörpern beschicktes Kontaktrohr geführt wird, der Umsatz der Reaktanden ≤ 5 mol-%, meist ≤ 2 mol-% beträgt) Trägerkörper mit pulverförmiger Aktivmasse oder mit pulverförmiger Vorläufermasse zu beschichten, kann man den Trägerkörper in vielen Fällen auch mit einer Lösung der katalytisch aktiven Substanz oder mit einer Lösung einer Vorläufersubstanz tränken und nachfolgend das Lösungsmittel verflüchtigen und gegebenenfalls eine chemische Reduktion und/oder thermische Behandlung (gegebenenfalls in molekularen Sauerstoff enthaltender Atmosphäre) anschließen. Die auf dieser Weise resultierenden geometrischen Katalysatorformkörper werden üblicherweise als Träger- oder Tränkkatalysatoren bezeichnet.

Unter der Längstausdehnung L solcher geometrischer Katalysatorformkörper (wie ganz generell von geometrischen Formkörpern in dieser Schrift) wird die längste mögliche direkte Verbindungslinie zweier auf der Oberfläche des Katalysatorformkörpers befindlicher Punkte verstanden. Sie beträgt (auch bei geometrischen Inertformkörpern) meist 1 bis 20 mm, oft 2 bis 15 mm und vielfach 3 bzw. 4 bis 10 bzw. bis 8 oder bis 6 mm. Im Fall von Ringen liegt die Wanddicke darüber hinaus üblicherweise bei 0,5 bis 6 mm, häufig bei 1 bis 4 bzw. bis 3 oder bis 2 mm.

Nicht bei allen heterogen katalysierten partiellen Gasphasenoxidationen am in den Rohren von Rohrbündelreaktoren befindlichen Katalysatorfestbett besteht das Katalysatorfestbett aus einer längs des individuellen Kontaktrohres einheitlichen Schüttung aus einer einzigen Sorte geometrischer Katalysatorformkörper. Vielmehr kann das Katalysatorfestbett über die Gesamtlänge des Kontaktrohres auch aus einem homogenisierten Gemisch mehrerer (d. h., wenigstens zwei) voneinander unterscheidbarer Sorten $S^i$ von geometrischen Katalysatorformkörpern oder von geometrischen Katalysatorformkörpern und geometrischen Inertformkörpern bestehen (d. h., ein solches Gemisch kann aus wenigstens zwei voneinander unterscheidbaren Sorten von geometrischen Katalysatorformkörpern, oder aus einer einzigen Sorte von geometrischen Katalysatorformkörpern und aus einer einzigen Sorte von geometrischen Inertformkörpern, oder aus wenigstens zwei Sorten von voneinander unterscheidbaren geometrischen Katalysatorformkörpern und einer einzigen Sorte von geometrischen Inertformkörpern, oder aus wenigstens zwei Sorten von voneinander unterscheidbaren geometrischen Katalysatorformkörpern und wenigstens zwei Sorten von voneinander unterscheidbaren geometrischen Inertformkörpern bestehen). Mögliche Unterscheidungsmerkmale der voneinander verschiedenen Sorten $S^i$ sind die Art der Geometrie, die Art der Aktivmasse, die Art des Trägermaterials etc.. Als Materialien für die geometrischen Inertformkörper kommen die gleichen Materialien in Betracht, die bereits für die inerten geometrischen Trägerformkörper bei den Schalenkatalysatoren empfohlen wurden und in den Ablauf der Gasphasenpartialoxidation im wesentlichen nicht eingreifen. Grundsätzlich kommen alle inerten Trägerformkörper auch als geometrische Inertformkörper zur Verdünnung von geometrischen Katalysatorformkörpern in einem Katalysatorfestbett in Betracht. Durch eine solche Verdünnung kann die volumenspezifische Aktivität eines Katalysatorfestbetts auf den Bedarf der jeweiligen heterogen katalysierten partiellen Gasphasenoxidation spezifisch eingestellt werden.

Der Wortlaut "homogenisiertes Gemisch" meint dabei, dass Maßnahmen ergriffen worden sind, um die voneinander verschiedenen Sorten geometrischer Formkörper (bzw. die verschiedenen Längstausdehnungen innerhalb einer Sorte) miteinander homogen zu vermengen. Im Idealfall erreicht die homogene Vermengung entlang des gesamten Längsabschnitts den statistischen Durchschnitt und dies auch bezüglich der jeweiligen individuellen Sorte.

Vielfach besteht eine Kontaktrohrbeschickung (eine Kontaktrohrbefüllung) mit einem Katalysatorfestbett aber auch aus mehreren voneinander unterscheidbar übereinander (hintereinander) angebrachten Längsabschnitten (Katalysatorfestbett(längs)abschnitten, Katalysatorschüttungsabschnitten). Jeder einzelne Längsabschnitt kann dabei über seine Länge einheitlich so gestaltet werden, wie es für ein über seine gesamte Kontaktrohrlänge einheitlich beschicktes Kontaktrohr bereits ausgeführt wurde. Beim Übergang von einem in sich einheitlichen Schüttungsabschnitt zum nächsten in sich einheitlichen Schüttungsabschnitt ändert sich die Zusammenstellung (Zusammensetzung) der Schüttung abrupt.

Es entstehen so längs eines individuellen Kontaktrohres Katalysatorfestbettschüttungen, die eine heterogene Struktur aufweisen. Man spricht auch von einer strukturierten Befüllung (bzw. Schüttung) der Kontaktrohre. Am Anfang (in Strömungsrichtung des das Kontaktrohr durchströmenden Reaktionsgases geblickt) und/oder am Ende des Kontaktrohres wird das Katalysatorfestbett häufig durch eine alleinige Schüttung aus geometrischen Inertformkörpern abgeschlossen.

Beispiele für solche strukturierten Befüllungen von Kontaktrohren sind unter anderem in den Schriften US 2006/0161019, EP-A 979 813, EP-A 090 744, EP-A 456 837, EP-A 1 106 598, US 5,198,581 und US 4,203,903 beschrieben.

In der Regel wird die Befüllung eines Kontaktrohres mit einem strukturierten Katalysatorfestbett so gestaltet, dass die volumenspezifische Aktivität des Katalysatorfestbetts in Strömungsrichtung des Katalysatorfestbetts zunimmt. Die volumenspezifische Aktivität eines in sich einheitlichen Längsabschnittes einer Katalysatorfestbettbeschickung eines Kontaktrohres ist dann erhöht, wenn bei durchgehender Beschickung des Kontaktrohres wie im entsprechenden Längsabschnitt des Kontaktrohres unter ansonsten identischen Reaktionsbedingungen (d. h., identische Zusammensetzung des Reaktionsgasgemischs, identische Belastung der Katalysatorfestbettbeschickung mit Reaktionsgasgemisch sowie identische Eintrittstemperatur des Wärmeträgers und identische Strömungsbedingungen des Wärmeträgers) ein erhöhter Eduktumsatz (bezogen auf den einmaligen Durchgang des Reaktionsgasgemischs durch das Kontaktrohr) resultiert.

Unter der Belastung eines einen Reaktionsschritt katalysierenden Katalysatorfestbetts mit Reaktionsgas oder mit einer Reaktionsgaskomponente wird dabei die Menge an Reaktionsgas bzw. an Reaktionsgaskomponente in Normlitern (= Nl; das Volumen in Litern, das die entsprechende Reaktionsgas- bzw. Reaktionsgaskomponentenmenge bei Normalbedingungen, d. h., bei 25 °C und 1bar, einnehmen würde) verstanden, die pro Stunde durch einen Liter Katalysatorfestbett geführt wird. Reine Inertmaterialschüttungsabschnitte werden dabei nicht miteinbezogen.

Gemäß der Lehre des Standes der Technik sollen für eine heterogen katalysierte partielle Gasphasenoxidation einer organischen Verbindung die geometrischen Abmessungen einer Sorte von geometrischen Katalysatorformkörpern bzw. einer Sorte von geometrischen Inertformkörpern, die zur Beschickung eines Längsabschnitts eines Kontaktrohres mit einem einheitlichen Katalysatorfestbett verwendet werden, innerhalb der jeweiligen Sorte möglichst einheitlich sein (vgl. US 2006/0205978 und WO 2005/113123).

Eigene Untersuchungen haben jedoch ergeben, dass sich eine definierte Uneinheitlichkeit der vorgenannten Abmessungen auf die Selektivität der Zielproduktbildung vorteilhaft auswirkt.

Demgemäß stellt vorliegende Erfindung ein Verfahren zur Beschickung eines Längsabschnitts eines Kontaktrohres gemäß Anspruch 1, ein Verfahren zur Beschickung eines Kontaktrohres gemäß Anspruch 4, einen Rohrbündelreaktor gemäß Anspruch 5, und ein Verfahren zur heterogen katalysierten partiellen Gasphasenoxidation einer organischen Verbindung gemäß Anspruch 6.

Erfindungsgemäß bevorzugt weisen weniger als 3 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen erfindungsgemäß bevorzugt weniger als 3 % der Gesamtzahl der zu $S^i$ gehörigen geometrischen Formkörper eine Längstausdehnung $L_S^i$ auf, für die gilt $1{,}10 \cdot D_S^i < L_S^i$.

Erfindungsgemäß ganz besonders bevorzugt weisen weniger als 1 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen erfindungsgemäß ganz besonders bevorzugt weniger als 1 % der Gesamtzahl der zu $S^i$ gehörigen geometrischen Formkörper eine Längstausdehnung $L_S^i$ auf, für die gilt $1{,}10 \cdot D_S^i < L_S^i$.

Mit Vorteil sind die vorgenannten Bedingungen (Maßgaben) für die Mehrzahl und mit besonderem Vorteil für jede der verschiedenen Sorten $S^i$ innerhalb des Katalysatorfestbettabschnitts erfüllt.

Mit besonderem Vorteil ist das erfindungsgemäße Verfahren dadurch gekennzeichnet, dass wenigstens innerhalb einer Sorte $S^i$ von geometrischen Formkörpern des Katalysatorfestbettabschnitts die Maßgabe M*, dass

| 50 bis 60 % | (vorzugsweise 55 %) der Gesamtzahl der zu $S^i$ gehörigen geometrischen Formkörper eine Längstausdehnung $L_S^i$ aufweisen, für die gilt $0{,}98 \cdot D_S^i \leq L_S^i \leq 1{,}02 \cdot D_S^i$, |
| wenigstens 15% | der Gesamtzahl der zu $S^i$ gehörigen geometrischen Formkörper eine Längstausdehnung $L_S^i$ aufweisen, für die gilt $0{,}94 \cdot D_S^i \leq L_S^i < 0{,}98 \cdot D_S^i$, |
| wenigstens 15 % | der Gesamtzahl der zu $S^i$ gehörigen geometrischen Formkörper eine Längstausdehnung $L_S^i$ aufweisen, für die gilt $1{,}02 \cdot D_S^i < L_S^i \leq 1{,}10 \cdot D_S^i$, |
| weniger als 5 % | der Gesamtzahl der zu $S^i$ gehörigen geometrischen Formkörper eine Längstausdehnung $L_S^i$ aufweisen, für die gilt $0{,}94 \cdot D_S^i > L_S^i$, und |

| weniger als 5 % | der Gesamtzahl der zu $S^i$ gehörigen geometrischen Formkörper eine Längstausdehnung $L_S^i$ aufweisen, für die gilt |
| --- | --- |
| | $1{,}10 \cdot D_S^i < L_S^i,$ |

erfüllt ist.

Erfindungsgemäß bevorzugt weisen im vorstehenden Raster weniger als 3 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen im vorstehenden Raster vorteilhaft weniger als 3 % der Gesamtzahl der zu $S^i$ gehörigen geometrischen Formkörper eine Längstausdehnung $L_S^i$ auf, für die gilt $1{,}10 \cdot D_S^i < L_S^i$.

Erfindungsgemäß ganz besonders bevorzugt weisen im vorstehenden Raster weniger als 1 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen im vorstehenden Raster vorzugsweise weniger als 1 % der Gesamtzahl der zu $S^i$ gehörigen geometrischen Formkörper eine Längstausdehnung $L_S^i$ auf, für die gilt $1{,}10 \cdot D_S^i < L_S^i$.

Mit Vorteil sind die vorgenannten Rasterbedingungen (Maßgaben) für die Mehrzahl und mit besonderem Vorteil für jede der verschiedenen Sorten $S^i$ innerhalb des Katalysatorfestbettabschnitts erfüllt.

Mit ganz besonderem Vorteil ist das erfindungsgemäße Verfahren dadurch gekennzeichnet, dass wenigstens innerhalb einer Sorte $S^i$ von geometrischen Formkörpern des Katalysatorfestbettabschnitts die Maßgabe M**, dass

| 50 bis 60 % | (vorzugsweise 55 %) der Gesamtzahl der zu $S^i$ gehörigen geometrischen Formkörper eine Längstausdehnung $L_S^i$ aufweisen, für die gilt |
| --- | --- |
| | $0{,}98 \cdot D_S^i \leq L_S^i \leq 1{,}02 \cdot D_S^i,$ |
| wenigstens 20 % | der Gesamtzahl der zu $S^i$ gehörigen geometrischen Formkörper eine Längstausdehnung $L_S^i$ aufweisen, für die gilt |
| | $0{,}94 \cdot D_S^i \leq L_S^i < 0{,}98 \cdot D_S^i,$ |
| wenigstens 20 % | der Gesamtzahl der zu $S^i$ gehörigen geometrischen Formkörper eine Längstausdehnung $L_S^i$ aufweisen, für die gilt |
| | $1{,}02 \cdot D_S^i < L_S^i \leq 1{,}10 \cdot D_S^i,$ |
| weniger als 5 % | der Gesamtzahl der zu $S^i$ gehörigen geometrischen Formkörper eine Längstausdehnung $L_S^i$ aufweisen, für die gilt |
| | $0{,}94 \cdot D_S^i > L_S^i,$ und |
| weniger als 5 % | der Gesamtzahl der zu $S^i$ gehörigen geometrischen Formkörper eine Längstausdehnung $L_S^i$ aufweisen, für die gilt |
| | $1{,}10 \cdot D_S^i < L_S^i,$ |

erfüllt ist.

Erfindungsgemäß bevorzugt weisen im vorstehenden Raster weniger als 3 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen im vorstehenden Raster mit Vorteil weniger als 3 % der Gesamtzahl der zu $S^i$ gehörigen geometrischen Formkörper eine Längstausdehnung $L_S^i$ auf, für die gilt $1{,}10 \cdot D_S^i < L_S^i$.

Erfindungsgemäß ganz besonders bevorzugt weisen im vorstehenden Raster weniger als 1 % der Gesamtzahl der zu $S^i$ gehörigen geometrischen Formkörper eine Längstausdehnung $L_S^i$ auf, für die gilt $0{,}94 \cdot D_S^i > L_S^i$.

Ferner weisen im vorstehenden Raster vorzugsweise weniger als 1 % der Gesamtzahl der zu $S^i$ gehörigen geometrischen Formkörper eine Längstausdehnung $L_S^i$ auf, für die gilt $1{,}10 \cdot D_S^i < L_S^i$.

Erfindungsgemäß sind die vorgenannten Rasterbedingungen (Maßgaben) für jede der verschiedenen Sorten $S^i$ innerhalb des Katalysatorfestbettabschnitts jeweils erfüllt.

Weiterhin ist es für alle in dieser Schrift aufgeführten Raster besonders vorteilhaft, wenn keine zu $S^i$ gehörigen geometrischen Formkörper eine Längstausdehnung $L_S^i$ aufweisen, für die gilt $0{,}94 \cdot D_S^i > L_S^i$.

Ferner ist es für alle in dieser Schrift aufgeführten Raster besonders vorteilhaft, wenn keine zu $S^i$ gehörigen geometrischen Formkörper eine Längstausdehnung $L_S^i$ aufweisen, für die gilt $1{,}10 \cdot D_S^i < L_S^i$.

Der Median $D_S^i$ der Längstausdehnungen $L_S^i$ der geometrischen Formkörper einer Sorte $S^i$ ist dabei so definiert, dass 50 % aller (der) Längstausdehnungen $L_S^i$ der geometrischen Formkörper einer Sorte $S^i$ kleiner oder gleich groß wie $D_S^i$ sind (wobei der Median, soweit in dieser Schrift nichts anderes explizit gesagt wird, immer über die in einem einheitlich beschickten Längsabschnitt des Kontaktrohres befindlichen jeweiligen Formkörper zu bilden ist).

Grundsätzlich kann sich ein einheitlich beschickter Längsabschnitt des Kontaktrohres beim erfindungsgemäßen Verfahren über die gesamte Kontaktrohrlänge erstrecken. Selbstverständlich kann das gesamte im Kontaktrohr befindliche Katalysatorfestbett aber auch aus mehreren voneinander unterscheidbaren (in sich jeweils einheitlich beschickten) Katalysatorfestbettabschnitten (Längsabschnitten) bestehen. In diesem Fall wird das erfindungsgemäße Verfahren auf jeden der verschiedenen Katalysatorfestbettabschnitte angewendet. Weist das Katalysatorfestbett in einem Kontaktrohr auch Längsabschnitte auf, die ausschließlich aus geometrischen Inertformkörpern bestehen, ist es vorteilhaft, wenn die erfindungsgemäße Verfahrensweise auch auf solche inerten Abschnitte angewendet wird (auch diese die erfindungsgemäßen Maßgaben erfüllen). Allerdings ist die Anwendung der erfindungsgemäßen Verfahrensweise auf solche inerten Abschnitte weniger relevant als im Fall von katalytisch aktiven Abschnitten (diese enthalten in jedem Fall katalytisch aktive geometrische Katalysatorformkörper).

Inerte Abschnitte können z. B. dazu verwendet werden, innerhalb eines Kontaktrohres katalytisch aktive Abschnitte voneinander räumlich zu trennen.

Im einfachsten, und anwendungstechnisch bevorzugten, Fall können sich voneinander verschiedene, in sich jedoch jeweils einheitliche, Katalysatorfestbett(längs)abschnitte (insbesondere die katalytisch aktiven) eines Kontaktrohres nur dadurch voneinander unterscheiden (wenigstens soweit sie den gleichen Reaktionsschritt katalysieren), dass eine einzige Sorte von Aktivmasse aufweisenden geometrischen Katalysatorformkörpern mit einem unterschiedlichen Anteil einer einzigen Sorte von inerten, keine Aktivmasse aufweisenden, geometrischen Inertformkörpern (im einfachsten Fall können diese, wie bereits gesagt, inerte Träger(form)körper sein; es kann sich aber auch um aus Metall (z. B. Edelstahl) bestehende Inertformkörper handeln) (in homogenisierter Form) verdünnt ist. Mit Vorteil unterscheiden sich alle den gleichen Reaktionsschritt katalysierenden Katalysatorfestbett (längs)abschnitte eines für eine heterogen katalysierte partielle Gasphasenoxidation mit einem Katalysatorfestbett beschickten Kontaktrohres ausschließlich in der vorgenannten Weise (dabei bilden der nur mit der einen Sorte von geometrischen Katalysatorformkörpern beschickte Katalysatorfestbettlängsabschnitt und der nur mit der einen Sorte von geometrischen Inertformkörpern beschickte Katalysatorfestbettlängsabschnitt die beiden möglichen Verdünnungsgrenzfälle). Reine Inertschüttungen können aber auch aus einer separaten Sorte von Inertformkörpern bestehen.

Die eine Sorte von inerten Verdünnungsformkörpern (Inertformkörpern) weisen die gleiche Geometrie wie die eine Sorte von katalytisch aktiven Katalysatorformkörpern auf.

Besteht ein einzelner erfindungsgemäßer Katalysatorfestbettabschnitt aus einem (homogenisierten) Gemisch nur einer Sorte von geometrischen Katalysatorformkörpern und nur einer Sorte von geometrischen Inertformkörpern, ist es erfindungsgemäß von Vorteil (insbesondere im Fall gleicher Geometrien der beiden Formkörpersorten) wenn der Median $D_{Kat}$ der Längstausdehnungen der nur einen Sorte von geometrischen Katalysatorformkörpern und der Median $D_{Inert}$ der nur einen Sorte von geometrischen Inertformkörpern (gebildet über den Katalysatorfestbettabschnitt) von ähnlicher Größe sind. Anwendungstechnisch vorteilhaft ist es dabei, wenn $0{,}90 \leq D_{Kat}/D_{Inert} \leq 1{,}10$ erfüllt ist. Anwendungstechnisch ganz besonders vorteilhaft gilt für das Verhältnis der beiden Mediane dabei $0{,}95 \leq D_{Kat}/D_{Inert} \leq 1{,}05$. Am besten ist es, wenn $0{,}98 \leq D_{Kat}/D_{Inert} \leq 1{,}02$, bzw. $D_{Kat}/D_{Inert} = 1$ ist. Das Verhältnis $D_{Kat}/D_{Inert}$ soll im folgenden mit V abgekürzt werden.

Bestehen alle katalytisch aktiven Katalysatorfestbett(längs)abschnitte einer Katalysatorfestbettbeschickung eines Kontaktrohres aus unterschiedlichen (homogenisierten) Verdünnungsgraden (Gemischen) einer einzigen Sorte von geometrischen Katalysatorformkörpern mit einer einzigen Sorte von geometrischen Inertformkörpern (wenigstens soweit sie den selben Reaktionsschritt katalysieren), so sind die vorgenannten Verhältnisse anwendungstechnisch zweckmäßig in jedem einzelnen eine solche Verdünnung aufweisenden katalytisch aktiven Katalysatorfestbett(längs)abschnitt für sich erfüllt (erfindungsgemäß weisen dabei die beiden Formkörpersorten die gleiche Geometrie auf).

Anwendungstechnisch ganz besonders zweckmäßig (insbesondere dann, wenn die beiden Formkörpersorten die gleiche Geometrie aufweisen) liegt das Medianverhältnis in einem der vorgenannten Bereiche, wenn die Medianbildung über das gesamte innerhalb des Kontaktrohres befindliche katalytisch aktive Katalysatorfestbett (bzw. über alle den gleichen Reaktionsschritt katalysierenden Katalysatorfestbett(längs)abschnitte) erfolgt (noch besser liegt das Medianverhältnis in einem der vorgenannten Bereiche, wenn in die Medianbildung über das gesamte im Kontaktrohr befindliche Festbett auch reine Inertschüttungen einbezogen werden).

Durch eine Aneinanderreihung solcher, einen unterschiedlichen Verdünnungsgrad aufweisender, Katalysatorfestbett(längs)abschnitte (gebildet aus nur einer Sorte von geometrischen Inertformkörpern und nur einer Sorte von geometrischen Katalysatorformkörpern) kann man, jeweils spezifisch an die Erfordernisse der auszuführenden heterogen katalysierten partiellen Gasphasenoxidation angepasst, längs eines Kontaktrohres Verdünnungsprofile (Verdünnungsstrukturen) der unterschiedlichsten Art erzeugen, wobei die beiden Formkörpersorten anwendungstechnisch vorteilhaft die gleiche Geometrie aufweisen. In vielen Fällen wird die Verdünnungsstruktur so gewählt, dass der Verdünnungsgrad in Strömungsrichtung des Reaktionsgasgemischs abnimmt (d. h., die volumenspezifische Aktivmasse nimmt in Strömungsrichtung zu; dort wo die Reaktandenkonzentration hoch ist, ist die volumenspezifische Aktivität niedrig und umgekehrt). Bei Bedarf kann das Verdünnungsprofil (die Aktivitätsstrukturierung) aber auch umgekehrt oder völlig anders gewählt werden.

Wie bereits erwähnt, werden erfindungsgemäß alle katalytisch aktiven (sie enthalten in jedem Fall geometrische Katalysatorformkörper) Katalysatorfestbett(längs)abschnitte einer Katalysatorfestbettbeschickung eines Kontaktrohres (wenigstens soweit sie den gleichen Reaktionsschritt katalysieren) aus unterschiedlichen (homogenisierten) Verdünnungsgraden (Gemischen) einer einzigen Sorte von geometrischen Katalysatorformkörpern mit einer einzigen Sorte von geometrischen Inertformkörpern gebildet (einschließlich des Verdünnungsgrades "0"; ein solcher katalytisch aktiver Katalysatorfestbett(längs)abschnitt besteht ausschließlich aus der einen Sorte von geometrischen Katalysatorformkörpern).

Weisen die eine Sorte geometrischer Katalysatorformkörper und die eine Sorte geometrischer Inertformkörper mit Vorteil darüber hinaus die gleiche Geometrie auf und bildet man einen gemeinsamen Median $D^{Inert}_{Kat}$ über alle (über die Gesamtzahl G der) Längstausdehnungen $L_{Kat}$ und $L_{Inert}$ der geometrischen Katalysatorformkörper und der geometrischen Inertformkörper die sich in der Gesamtheit dieser Katalysatorfestbett(längs)abschnitte befinden, dann ist es erfindungsgemäß vorteilhaft, wenn die Maßgabe $M^G$, dass

| | |
|---|---|
| 40 bis 70 | (vorzugsweise 50 bis 60) % der Gesamtzahl G aus geometrischen Katalysatorformkörpern und geometrischen Inertformkörpern eine Längstausdehnung $L_{Kat,Inert}$ aufweisen, für die gilt $0{,}98 \cdot D^{Inert}_{Kat} \leq L_{Kat,Inert} \leq 1{,}02 \cdot D^{Inert}_{Kat}$, |
| wenigstens 10 | (vorzugsweise 15 bzw. 20) % der Gesamtzahl G eine Längstausdehnung $L_{Kat,Inert}$ aufweisen, für die gilt $0{,}94 \cdot D^{Inert}_{Kat} \leq L_{Kat,Inert} < 0{,}98 \cdot D^{Inert}_{Kat}$, |
| wenigstens 10 | (vorzugsweise 15 bzw. 20) % der Gesamtzahl G eine Längstausdehnung $L_{Kat,Inert}$ aufweisen, für die gilt $1{,}02 \cdot D^{Inert}_{Kat} < L_{Kat,Inert} \leq 1{,}10 \cdot D^{Inert}_{Kat}$, |
| weniger als 5 | (vorzugsweise weniger als 3 bzw. 1 (oder 0 %) % der Gesamtzahl G eine Längstausdehnung $L_{Kat,Inert}$ aufweisen, für die gilt $0{,}94 \cdot D^{Inert}_{Kat} > L_{Kat,Inert}$, und |
| weniger als 5 | (vorzugsweise weniger als 3 bzw. 1 (oder 0 %) % der Gesamtzahl G eine Längstausdehnung $L_{Kat,Inert}$ aufweisen, für die gilt $1{,}10 \cdot D^{Inert}_{Kat} < L_{Kat,Inert}$, |

erfüllt ist.

Es ist aber auch schon vorteilhaft, wenn die Maßgabe $M^G$ nur innerhalb eines einheitlichen Katalysatorfestbett(längs)abschnitts oder wenigstens über die Mehrzahl der Katalysatorfestbett(längs)abschnitte gebildet erfüllt wird.

Normalerweise folgen in einem Kontaktrohr diejenigen Katalysatorfestbett(längs)abschnitte, die den gleichen Reaktionsschritt katalysieren, in Strömungsrichtung des Katalysatorfestbetts unmittelbar aufeinander.

Wird in einem Kontaktrohr mehr (in der Mehrzahl der Fälle wird in einem Kontaktrohr nur ein Reaktionsschritt katalysiert) als ein Reaktionsschritt katalysiert (z. B. zunächst der Schritt von Propylen zu Acrolein und daran anschließend in Strömungsrichtung der Schritt von Acrolein zu Acrylsäure), so weist das Katalysatorfestbett in der Regel eine der Anzahl der Reaktionsschritte entsprechende Anzahl von vorgenannten Katalysatorfestbett(längs)abschnittsequenzen auf. Beginnt oder endet eine solche Katalysatorfestbett(längs)abschnittsequenz mit einem nur aus Inertformkörpern bestehenden Festbettabschnitt, ist es erfindungsgemäß günstig, wenn diese Inertformkörper von der gleichen Sorte sind, wie die in der nachfolgenden bzw. vorausgehenden Katalysatorfestbett(längs)abschnittsequenz verwendeten. Ferner ist es erfindungsgemäß vorteilhaft, wenn das vorgenannte Beziehungsgeflecht (die vorgenannten Maßgaben $M^G$) auch dann erfüllt ist, wenn man solche nur aus Inertformkörpern bestehende Festbettabschnitte einbezieht.

Zur Herstellung einer Sorte $S^i$ an geometrischen Schalenkatalysatorformkörpern (Trägerkatalysatorformkörpern), die das erfindungsgemäße Anforderungsprofil erfüllt, wird man in der Regel von einer Sorte von geometrischen Trägerformkörpern ausgehen, die (als eine Sorte geometrischer Inertformkörper betrachtet) bereits als solche das erfindungsgemäße Anforderungsprofil erfüllt, und diese nach den bekannten Verfahren des Standes der Technik mit feinteiliger Aktivmasse bzw. mit feinteiliger Vorläufermasse der Aktivmasse einheitlich beschichten (bzw. tränken). Zu diesem Zweck kann beispielsweise das in der US 2006/0205978 beschriebene Beschichtungsverfahren angewendet werden. Alternativ kann das Beschichtungsverfahren der EP-A 714 700 angewendet werden.

Um eine Sorte von geometrischen Trägerformkörpern zu erzeugen, die bezüglich ihrer Längstausdehnungen das erfindungsgemäße Anforderungsprofil erfüllt, kann in einfacher Weise von geometrischen Trägerformkörpersorten ausgegangen werden, für die zwischen dem Median ihrer Längstausdehnungen $D_S^*$ und den zugehörigen Längstausdehnungen $L_S^*$ die Beziehung B

$$0{,}99 \cdot D_S^* \leq L_S^* \leq 1{,}01 \cdot D_S^* \qquad \text{(B)},$$

erfüllt ist.

In der erforderlichen Weise voneinander verschiedene Trägerformkörpersorten können dann in den erforderlichen Mengenverhältnissen miteinander homogen vermengt (homogenisiert) werden. In entsprechender Weise sind erfindungsgemäß geeignete Sorten $S^i$ von geometrischen Inertformkörpern erhältlich.

Zur Herstellung einer Sorte $S^i$ an geometrischen Vollkatalysatorformkörpern, die das erfindungsgemäße Anforderungsprofil erfüllt, kann in entsprechender Weise vorgegangen werden. D. h., z. B. nach der in der US 2005/0263926 offenbarten Verfahrensweise werden Vollkatalysatorformkörpersorten (bzw. noch zu calcinierende (thermisch zu behandelnde) Vollkatalysatorvorläuferformkörpersorten) erzeugt, die die Beziehung B erfüllen. Durch entsprechendes (homogenisierendes) Abmischen solcher voneinander verschiedener Sorten können nachfolgend erfindungsgemäß geforderte Sorten $S^i$ erzeugt werden.

Alles in dieser Schrift gesagte gilt insbesondere dann, da es sich sowohl bei den geometrischen Katalysatorformkörpersorten als auch bei den geometrischen Inertformkörpersorten um Ringe handelt.

Dies gilt insbesondere dann, wenn die Aktivmasse derartiger Katalysatorringe ein Multielementoxid der allgemeinen Formel I,

$$Mo_{12}Bi_aFe_bX^1_cX^2_dX^3_eX^4_fO_n \qquad (I),$$

mit

X$^1$ = Nickel und/oder Kobalt,
X$^2$ = Thallium, ein Alkalimetall und/oder ein Erdalkalimetall,
X$^3$ = Zink, Phosphor, Arsen, Bor, Antimon, Zinn, Cer, Blei, Vanadium, Chrom und/oder Wolfram,
X$^4$ = Silizium, Aluminium, Titan und/oder Zirkonium,
a = 0,2 bis 5,
b = 0,01 bis 5,
c = 0 bis 10,
d = 0 bis 2,
e = 0 bis 8,
f = 0 bis 10, und
n = eine Zahl, die durch die Wertigkeit und Häufigkeit der von Sauerstoff verschiedenen Elemente in I bestimmt wird,

ist (selbstverständlich können vorgenannte Multielementoxide I aber auch als Aktivmasse für alle anderen möglichen geometrischen Katalysatorformkörperarten verwendet werden.)

Die Herstellung entsprechender Vollkatalysatorringe sowie Schalenkatalysatorringe findet sich z. B. beschrieben in der WO 02/30569, in der WO 2005/030393, in Research Disclosure RD 2005-497012, in der DE-A 10 2007 005 602 sowie in der DE-A 10 2007 004 961. In den vorgenannten Schriften werden solche ringförmigen Katalysatoren (sowie ganz allgemein Katalysatoren mit einem Multielementoxid I als Aktivmasse), deren Aktivmasse ein Multielementoxid I ist, insbesondere für eine heterogen katalysierte Partialoxidation von Propylen zu Acrolein bzw. Acrolein und Acrylsäure sowie von iso-Buten zu Methacrolein empfohlen. Die dabei in vorgenannten Schriften empfohlenen Ringgeometrien sind im Sinn der vorliegenden Erfindung als Median-Ringgeometrie einer Sorte ringförmiger Katalysatorformkörper zu verstehen. D.h., der Median der Ringinnendurchmesser, der Median der Ringaußendurchmesser und der Median der Ringlänge einer erfindungsgemäß zu verwendenden ringförmigen Katalysatorformkörpersorte $S^i$ kann die in den vorgenannten Schriften jeweils genannten Ausmaße aufweisen.

Der Außendurchmesser dieser Median-Ringgeometrien kann z. B. 2 bis 10 mm, oder 2 bis 8 mm, bzw. 4 bis 8 mm betragen.

Die Länge dieser Median-Ringgeometrien kann ebenfalls z. B. 2 bis 10 mm, oder 2 bis 8 mm, bzw. 4 bis 8 mm betragen. Der Median der Wandstärke solcher Ringgeometrien beträgt in zweckmäßiger Weise in der Regel 1 bis 3 mm.

Der Median der jeweiligen Ringabmessung (dies gilt so auch im Fall aller anderen in dieser Schrift angesprochenen Ringgeometrien) einer Sorte $S^i$ von Katalysatorformkörpern in Bezug auf den Median einer speziellen Abmessung der jeweiligen Geometrie und den Einzelwerten dieser Abmessung aus der ihr Median gebildet wird) kann dabei zu den entsprechenden Einzelwerten dieser Abmessung, aus denen er gebildet wird, im selben Verhältnis stehen wie $L_S^i$ zu $D_S^i$ gemäß der vorliegenden Erfindung.

Wird in dieser Schrift von gleichen Geometrien verschiedener Sorten geometrischer Formkörper gesprochen, so ist damit gemeint, dass die unterschiedlichen Sorten geometrischer Formkörper im wesentlichen die gleiche Median-Geometrie aufweisen. D.h., die Mediane von einander entsprechenden Einzelabmessungen der Formkörpergeometrie unterscheiden sich, bezogen auf den arithmetischen Mittelwert der beiden Mediane, um weniger als 10 %, vorzugsweise um weniger als 5 %. Die Einzelabmessungen einer Median-Geometrie können dabei grundsätzlich die im Stand der Technik empfohlenen Werte für die entsprechende Abmessung einer Individualgeometrie aufweisen.

Eine besonders bevorzugte Median-Ringgeometrie für Multimetalloxid (I)-Vollkatalysatorformkörper ist z.B. die Geometrie 5 mm Außendurchmesser A x 3 mm Länge L x 2 mm Innendurchmesser I (die im Stand der Technik bereits als bevorzugte Individualgeometrie empfohlen wird).

Andere günstige Multimetalloxid (I)-Vollkatalysator-Median-Ringgeometrien A x L x I sind die Geometrien 5 mm x 2 mm x 2 mm, oder 5 mm x 3 mm x 3 mm, oder 5,5 mm x 3 mm x 3,5 mm, oder 6 mm x 3 mm x 4 mm, oder 6,5 mm x 3 mm x 4,5 mm, oder 7 mm x 3 mm x 5 mm, oder 7 mm x 7 mm x 3 mm, oder 7 mm x 7 mm x 4 mm.

Alle diese Multimetalloxid (I)-Vollkatalysator-Median-Ringgeometrien eignen sich z. B. sowohl für die gasphasenkatalytische Partialoxidation von Propylen zu Acrolein als auch für die gasphasenkatalytische Partialoxidation von iso-Buten oder tert-Butanol oder dem Methylether des tert.-Butanols zu Methacrolein.

Betreffend die Aktivmassen der Stöchiometrie der allgemeinen Formel I betragen der stöchiometrische Koeffizient b vorzugsweise 2 bis 4, der stöchiometrische Koeffizient c vorzugsweise 3 bis 10, der stöchiometrische Koeffizient d vorzugsweise 0,02 bis 2, der stöchiometrische Koeffizient e vorzugsweise 0 bis 5 und der stöchiometrische Koeffizient f vorteilhaft 0,5 oder 1 bis 10. Besonders bevorzugt liegen die vorgenannten stöchiometrischen Koeffizienten gleichzeitig in den genannten Vorzugsbereichen.

Ferner ist $X^1$ vorzugsweise Kobalt, $X^2$ ist vorzugsweise K, Cs und/oder Sr, besonders bevorzugt K, $X^3$ ist bevorzugt Wolfram, Zink und/oder Phosphor und $X^4$ ist bevorzugt Si. Besonders bevorzugt weisen die Variablen $X^1$ bis $X^4$ gleichzeitig die vorgenannten Bedeutungen auf.

Das über die Median-Geometrien der Katalysatorformkörper gesagte gilt in entsprechender Weise für die Inertformkörper. Vorzugsweise sind die Inertformkörper aus Steatit C 220 der Fa. CeramTec gefertigt.

Ringförmige Katalysatorformkörper werden anwendungstechnisch zweckmäßig mit ringförmigen Inertformkörpern verdünnt, um eine Aktivitätsstrukturierung der Katalysatorbeschickung im Kontaktrohr zu bewirken. Vorzugsweise weisen die ringförmigen Inertformkörper dabei die gleiche Median-Ringgeometrie wie die ringförmigen Katalysatorformkörper auf.

Für eine heterogen katalysierte partielle Gasphasenoxidation zur Herstellung von Acrolein oder Methacrolein wird die Katalysatorbeschickung im Kontaktrohr mit den vorstehend beschriebenen ringförmigen Formkörpern vorzugsweise entweder auf der gesamten Länge des Kontaktrohres einheitlich mit nur einer erfindungsgemäßen Sorte $S^i$ von Vollkatalysatorringen gestaltet oder wie folgt strukturiert.

Am Kontaktrohreingang (in Strömungsrichtung des Reaktionsgasgemischs) wird auf einer Länge von 10 bis 60 %, bevorzugt 10 bis 50 %, besonders bevorzugt 20 bis 40 % und ganz besonders bevorzugt 25 bis 35 % (d.h., z. B. auf einer Länge von 0,70 bis 1,50 m, bevorzugt 0,90 bis 1,20 m), jeweils der Gesamtlänge der katalytisch aktiven Katalysatorbeschickung im Kontaktrohr, ein homogenisiertes Gemisch aus nur einer Sorte $S^i$ der vorgenannten ringförmigen Vollkatalysatoren und nur einer Sorte $S^j$ von ringförmigen Inertformkörpern (beide Formkörpersorten weisen vorzugsweise die gleiche Ringgeometrie auf) platziert, wobei der Gewichtsanteil der Verdünnungsformkörper (die Massendichten von Katalysatorformkörpern und Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 1,00 bis 3,00 m oder von 1,00 bis 2,70 m, bevorzugt 1,40 bis 3,00 m oder 2,00 bis 3,00 m) entweder eine nur in geringerem Umfang (als im ersten Abschnitt) verdünnte Schüttung der nur einen Sorte $S^i$ von ringförmigen Vollkatalysatoren mit der nur einen Sorte $S^j$ von ringförmigen Inertformkörpern, oder, ganz besonders bevorzugt, eine alleinige (unverdünnte) Schüttung derselben nur einen Sorte $S^i$ an ringförmigem Vollkatalysator. Natürlich kann auch über die gesamte Kontaktrohrlänge eine einheitliche Verdünnung gewählt werden.

Im übrigen kann die heterogen katalysierte partielle Gasphasenoxidation des Propylens zu Acrolein oder des iso-Butens zu Methacrolein in einem eine oder mehrere Temperaturzonen aufweisenden Rohrbündelreaktor so durchgeführt werden, wie es im Stand der Technik beschrieben ist (vgl. z.B. WO 2005/03093, DE-A 10 2007 005 602 sowie DE-A 10 2004 025 445 und den in diesen Schriften zitierten Stand der Technik). Als Aktivmasse für die geometrischen Katalysatorformkörper einer erfindungsgemäßen Kontaktrohrbeschickung kommen ferner Multielementoxide der allgemeinen Formel II,

$$Mo_{12}P_aV_bX^1{}_cX^2{}_dX^3{}_eSb_fRe_gS_hO_n \qquad (II),$$

mit

X$^1$ = Kalium, Rubidium und/oder Cäsium,
X$^2$ = Kupfer und/oder Silber,
X$^3$ = Cer, Bor, Zirkonium, Mangan und/oder Wismut,
a = 0,5 bis 3,
b = 0,01 bis 3,
c = 0,2 bis 3,

d = 0,01 bis 2,

e = 0 bis 2,

f = 0,01 bis 2,

g = 0 bis 1,

h = 0,001 bis 0,5, und

n = eine Zahl, die durch die Wertigkeit und Häufigkeit der von Sauerstoff verschiedenen Elemente in II bestimmt wird,

in Betracht.

Solche geometrischen Katalysatorformkörper eignen sich mit Vorteil insbesondere für eine heterogen katalysierte partielle Gasphasenoxidation von Methacrolein zu Methacrylsäure.

Bevorzugt sind vorgenannte Katalysatorformkörper ebenfalls ringförmige Vollkatalysatoren wie sie z.B. nach der in der EP-A 467 144 beschriebenen Verfahrensweise erhältlich sind. Als Median-Ringgeometrien kommen dabei insbesondere die in der EP-A 467 144 sowie die bezüglich der Multielementoxide I in dieser Schrift empfohlenen Individualgeometrien in Betracht. Eine bevorzugte Median-Ringgeometrie ist diejenige mit A x L x l = 7 mm x 7 mm x 3 mm (vgl. auch DE-A 10 2007 005 602).

Eine strukturierte Verdünnung mit ringförmigen Inertformkörpern kann z. B. wie für den Fall der heterogen katalysierten Partialoxidation von Propylen zu Acrolein beschrieben erfolgen. Im übrigen können die in der EP-A 467 144 sowie der DE-A 10 2007 005 602 beschriebenen Partialoxidationsverfahrensbedingungen angewendet werden. Für die heterogen katalysierte partielle Gasphasenoxidation von Kohlenwasserstoffen mit mindestens vier Kohlenstoffatomen (insbesondere n-Butan, n-Butene und/oder Benzol) zu Maleinsäureanhydrid kommen mit Vorteil Multielementoxidaktivmassen der allgemeinen Formel III,

$$V_1P_bFe_cX^1{}_dX^2{}_eO_n \qquad (III),$$

in der die Variablen folgende Bedeutung aufweisen:

$X^1 =$ Mo, Bi, Co, Ni, Si, Zn, Hf, Zr, Ti, Cr, Mn, Cu, B, Sn und/oder Nb,

$X^2 =$ K, Na, Rb, Cs und/oder Tl,

b = 0,9 bis 1,5,

c = 0 bis 0,1,

d = 0 bis 0,1,

e = 0 bis 0,1, und

n = eine Zahl, die durch die Wertigkeit und Häufigkeit der von Sauerstoff verschiedenen Elemente in III bestimmt wird,

für erfindungsgemäß zu verwendende geometrische Katalysatorformkörper in Betracht.

Mit Vorteil sind diese Katalysatorformkörper ebenfalls ringförmige Vollkatalysatoren wie sie z. B. gemäß der WO 03/078310, der WO 01/68245, der DE-A 10 2005 035 978 sowie der DE-A 10 2007 005 602 erhältlich sind. Als Median-Ringgeometrien kommen dabei insbesondere die in den vorgenannten Schriften sowie die bezüglich der Multielementoxide I in dieser Schrift empfohlenen Individualgeometrien in Betracht. Eine bevorzugte Median-Ringgeometrie ist diejenige mit A x L x l = 5 mm x 3,2 mm x 2,5 mm (vgl. auch DE-A 10 2007 005 602).

Eine strukturierte Verdünnung mit ringförmigen Inertformkörpern kann z. B. wie für den Fall der heterogen katalysierten Partialoxidation von Propylen zu Acrolein beschrieben erfolgen.

Im übrigen sind die in der WO 03/078 310, der WO 01/68245, der DE-A 10 2005 035 978 sowie der DE-A 10 2007 005 602 empfohlenen Partialoxidationsverfahrensbedingungen anwendbar.

Für die heterogen katalysierte partielle Gasphasenoxidation von Acrolein zu Acrylsäure kommen mit Vorteil Multielementoxidaktivmassen der allgemeinen Formel IV,

$$Mo_{12}V_aX^1{}_bX^2{}_cX^3{}_dX^4{}_eX^5{}_fX^6{}_gO_n \qquad (IV),$$

in der die Variablen folgende Bedeutung haben:

$X^1 =$ W, Nb, Ta, Cr und/oder Ce,

$X^2 =$ Cu, Ni, Co, Fe, Mn und/oder Zn,

$X^3 =$ Sb und/oder Bi,

$X^4 =$ eines oder mehrere Alkalimetalle (Li, Na, K, Rb, Cs) und/oder H,

$X^5 =$ eines oder mehrere Erdalkalimetalle (Mg, Ca, Sr, Ba),

$X^6 =$ Si, Al, Ti und/oder Zr,

a = 1 bis 6

b = 0,2 bis 4,

c = 0 bis 18, vorzugsweise 0,5 bis 18,

d = 0 bis 40,

e = 0 bis 2,

f = 0 bis 4,

g = 0 bis 40, und

n = eine Zahl, die durch die Wertigkeit und Häufigkeit der von Sauerstoff verschiedenen Elemente in IV bestimmt wird,

für erfindungsgemäß zu verwendende geometrische Katalysatorformkörper in Betracht.

Mit Vorteil sind diese Katalysatorformkörper ringförmige Schalenkatalysatoren, wie sie z.B. gemäß der DE-A 10 2004 025 445, der DE-A 10 350 822, der DE-A 10 2007 010 422, der US 2006/0205978 sowie der EP-A 714 700 und dem in diesen Schriften zitierten Stand der Technik erhältlich sind.

Als Median-Ringgeometrien kommen dabei insbesondere die in den vorgenannten Schriften empfohlenen Individualgeometrien in Betracht. Eine bevorzugte Median-Ringgeometrie ist diejenige mit A x L x I = 7 mm x 3 mm x 4 mm für die zugrunde liegenden ringförmigen Trägerformkörper.

Die Aktivmassenschalendicke kann 10 bis 1000 $\mu$m, bevorzugt 50 bis 500 $\mu$m und besonders bevorzugt 150 bis 250 $\mu$m betragen. Günstig sind die Schalendicken der beispielhaften Ausführungsformen der EP-A 714 700.

Für eine heterogen katalysierte partielle Gasphasenoxidation von Acrolein zu Acrylsäure wird die Katalysatorbeschickung im Kontaktrohr vorzugsweise entweder auf der gesamten Länge des Kontaktrohres einheitlich mit nur einer erfindungsgemäßen Sorte $S^i$ von Schalenkatalysatorringen gestaltet oder wie folgt strukturiert.

Am Kontaktrohreingang (in Strömungsrichtung des Reaktionsgasgemischs) wird auf einer Länge von 10 bis 60 %, bevorzugt 10 bis 50 %, besonders bevorzugt 20 bis 40 % und ganz besonders bevorzugt 25 bis 35 % (d.h., z. B. auf einer Länge von 0,70 bis 1,50 m, bevorzugt 0,90 bis 1,20 m), jeweils der Gesamtlänge der katalytisch aktiven Katalysatorbeschickung im Kontaktrohr, ein homogenisiertes Gemisch aus nur einer Sorte $S^i$ der oben genannten ringförmigen Schalenkatalysatoren und nur einer Sorte $S^j$ von ringförmigen Inertformkörpern (beide Formkörpersorten weisen vorzugsweise die gleiche Ringgeometrie auf) platziert, wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diesen ersten Beschickungsabschnitt befindet sich dann vorteilhaft bis zum Ende der Länge der Katalysatorbeschickung (d.h., z.B. auf einer Länge von 2,00 bis 3,00 m, bevorzugt 2,50 bis 3,00 m) entweder eine nur in geringerem Umfang (als im ersten Abschnitt) verdünnte Schüttung der nur einen Sorte $S^i$ von ringförmigen Vollkatalysatoren mit der nur einen Sorte $S^j$ von ringförmigen Inertformkörpern, oder, ganz besonders bevorzugt, eine alleinige (unverdünnte) Schüttung derselben nur einen Sorte $S^i$ an ringförmigem Schalenkatalysator.

Im übrigen kann die heterogen katalysierte partielle Gasphasenoxidation von Acrolein zu Acrylsäure in einem eine oder mehrere Temperaturzonen aufweisenden Rohrbündelreaktor so durchgeführt werden, wie es im Stand der Technik beschrieben ist (vgl. z.B. DE-A 10 2004 025 445, DE-A 10 350 822, DE-A 10 2007 010 422, US 2006/0205978 sowie EP-A 714 700 und der in diesen Schriften zitierte Stand der Technik).

Ein V und Sb enthaltendes Multielementoxid (insbesondere ein solches gemäß den Schriften US-A 6,528,683, oder US-A 6,586,361, oder US-A 6,362,345) eignet sich insbesondere für eine heterogen katalysierte partielle Oxidation von o-Xylol und/oder Naphthalin zu Phthalsäureanhydrid.

Vorzugsweise werden die vorgenannten Multielementoxide dabei als ringförmige Schalenkatalysatoren eingesetzt. Als Trägerkörper kommen dabei insbesondere solche in Betracht, die zu wenigstens 80 Gew.-% aus Titandioxid bestehen. Als beispielhafte Median-Ringgeometrien seien genannt die Ringgeometrien A x L x I = 8 mm x 6 mm x 5 mm, oder 8 mm x 6 mm x 4 mm, oder 8 mm x 6 mm x 3 mm und 7 mm x 7 mm x 4 mm.

Katalysatorformkörper, deren Aktivmasse elementares Silber auf einem oxidischen Trägerkörper aufweist, eignen sich (vor allem als Trägerkatalysatoren) insbesondere für eine heterogen katalysierte partielle Gasphasenoxidation von Ethylen zu Ethylenoxid (vgl. EP-A 496470).

Die Katalysatorformkörpergeometrie ist ringförmig. Als Trägerformkörper kommen vor allem solche in Betracht, die zu wenigstens 80 Gew.-% aus Aluminiumoxid (z. B. $Al_2O_3$) bestehen.

Als beispielhafte Median-Kugelgeometrie seien hier der Kugeldurchmesser 4 mm, 5 mm und 7 mm genannt.

Ganz generell kann bei den beschriebenen Verfahren der heterogen katalysierten partiellen Gasphasenoxidation eine reine Inertformkörperschüttung, deren Länge, bezogen auf die Gesamtlänge des Katalysatorfestbetts, im Kontaktrohr zweckmäßig 1 oder 5 bis 20 % beträgt, in Strömungsrichtung des Reaktionsgases das Katalysatorfestbett einleiten. Sie wird normalerweise als Aufheizzone für das Reaktionsgasgemisch genutzt.

Generell ist es bei dem erfindungsgemäßen Verfahren von Vorteil, wenn der Median $D_S^i$ der Längstausdehnung $L_S^i$ einer im Kontaktrohr zur Beschickung eines Katalysatorfestbettabschnitts mitverwendeten Sorte $S^i$ zum Innendruchmesser R des Kontaktrohres nachfolgendes Verhältnis aufweist: $R/D_S^i$ = 1,5 bis 5, vorzugsweise 2 bis 4 und besonders bevorzugt 3 bis 3,5.

Weiterhin ist es für das erfindungsgemäße Verfahren vorteilhaft, wenn der arithmetische Mittelwert $M_S^i$ der Längstausdehnungen $L_S^i$ die dem Median $D_S^i$ zugrunde liegen nicht mehr als 10 %, vorzugsweise nicht mehr als 5 % von $D_S^i$ abweicht (mit $D_S^i$ als Bezugsbasis). Alles in dieser Schrift gesagte gilt insbesondere dann, wenn es sich bei den geometrischen Katalysatorformkörpern und bei den geometrischen Inertformkörpern um Ringe handelt. Das Befüllen der Kontaktrohre kann ansonsten generell wie in der WO 2006/094 766 sowie der WO 2005/113 123 und der JP-A 2004 195 279 beschrieben erfolgen.

Alles in dieser Schrift gesagte gilt insbesondere auch für die eine Mo und V enthaltende Multielementoxidaktivmasse aufweisenden Schalenkatalysatoren der Schriften EP-A 1 254 707, EP-A 1 254 710, EP-A 1 254 709, WO 2004/035528, DE-A 102 48 584, DE-A 102 54 278, DE-A 102 54 279, WO 02/06199 und

WO 02/051539, sowie die durch diese Schalenkatalysatoren katalysierten Partialoxidationen von Propan zu Acrolein und/oder Acrylsäure sowie iso-Butan zu Methacrolein und/oder Methacrylsäure.

Weiterhin gilt alles in dieser Schrift gesagte auch für die eine Mo und V enthaltende Multielementoxidaktivmasse aufweisenden Schalenkatalysatoren der DE-A 10 2007 010 422 sowie die durch diese Schalenkatalysatoren katalysierten Partialoxidationen (insbesondere von Acrolein zu Acrylsäure).

Im übrigen beziehen sich alle Angaben in dieser Schrift, soweit nicht ausdrücklich etwas anderes gesagt wird, auf eine Temperatur von 25 °C und einen Druck von 1 atm. Beispiel und Vergleichsbeispiele

Vergleichsbeispiel 1

Wie in der WO 2005/030 393 der Vollkatalysator BVK 3 wurde unter Verwendung von TIMREX T 44 der Fa. Timcal AG (CH-Bodio) als Hilfsgraphit eine Sorte ringförmiger Vollkatalysatoren der Stöchiometrie (ohne Berücksichtigung von noch enthaltenem Graphit)

$$Mo_{12}Bi_1W_2Co_{5,5}Fe_{2,94}Si_{1,59}K_{0,08}O_x$$

hergestellt.

Die Mediangeometrie der ringförmigen Vollkatalysatoren war A x L X l = 5 mm x 3 mm x 2 mm.

Zwischen dem Median ihrer Längstausdehnung $D_S^i$ (5,83 mm) und den einzelnen Längstausdehnungen $L_S^i$ war erfüllt:

$$0,99 \cdot 5,83 \text{ mm} \leq L_S^i \leq 1,01 \cdot 5,83 \text{ mm}.$$

Ein Kontaktrohr (V2A Stahl; 21 mm Außendurchmesser, 3 mm Wandstärke, 15 mm Innendurchmesser, 100 cm Länge) wurde unter Mitverwendung von Steatit-Inertformkörperringen der gleichen ringförmigen Mediangeometrie in Strömungsrichtung des späteren Reaktionsgases wie folgt beschickt:

Abschnitt 1: 30 cm Länge, Schüttung nur aus den Inertformkörperringen;
Abschnitt 2: 70 cm Länge, Schüttung nur aus den ringförmigen Vollkatalysatoren.

Die Temperierung des Kontaktrohres erfolgte mittels eines mit Stickstoff geperlten Salzbades.

Das Kontaktohr wurde mit einem Beschickungsgasgemisch (Gemisch aus Luft, polymer grade Propylen und Stickstoff) der folgenden Zusammensetzung beschickt:

5 Vol.-% Propylen,
10 Vol.-% molekularer Sauerstoff und

als Restmenge bis 100 Vol.-% $N_2$.

Die Propylenbelastung des Katalysatorfestbetts wurde zu 50 Nl/(l•h) gewählt. Die Salzbadtemperatur wurde so eingestellt, dass der Propylenumsatz, bezogen auf einen einmaligen Durchgang des Reaktionsgasgemischs durch das Kontaktrohr, 95 mol-% betrug.

Die Selektivität der resultierenden Wertproduktbildung aus Acrolein und Acrylsäure betrug 95,7 mol-%.

Vergleichsbeispiel 2

Es wurde wie im Vergleichsbeispiel 1 verfahren. Zur Beschickung von Abschnitt 2 des Kontaktrohres wurde jedoch ein homogenisiertes Gemisch aus ringförmigen Vollkatalysatoren derselben Mediangeometrie und Aktivmassenzusammensetzung verwendet, wobei zwischen dem Median der Längstausdehnungen und den einzelnen Längstausdehnungen die folgenden Beziehungen erfüllt waren:

Für 80 % der Ringe: $0{,}98 \cdot 5{,}83 \text{ mm} \leq L_S^i \leq 1{,}02 \cdot 5{,}83 \text{ mm}$.
Für 20 % der Ringe: $1{,}02 \cdot 5{,}83 \text{ mm} < L_S^i \leq 1{,}10 \cdot 5{,}83 \text{ mm}$.

Die Selektivität der resultierenden Wertproduktbildung aus Acrolein und Acrylsäure betrug unter ansonsten identischen Betriebsbedingungen 95,8 mol-%.

Beispiel

Es wurde wie im Vergleichsbeispiel 1 verfahren. Zur Beschickung von Abschnitt 2 des Kontaktrohres wurde jedoch ein homogenisiertes Gemisch aus ringförmigen Vollkatalysatoren der gleichen Mediangeometrie und Aktivmassenzusammensetzung verwendet, wobei zwischen dem Median der Längstausdehnungen und den einzelnen Längstausdehnungen die folgenden Beziehungen erfüllt waren:

Für 60 % der Ringe: $0{,}98 \cdot 5{,}73 \text{ mm} \leq L_S^i \leq 1{,}02 \cdot 5{,}73 \text{ mm}$.
Für 20 % der Ringe: $0{,}94 \cdot 5{,}73 \text{ mm} \leq L_S^i < 0{,}98 \cdot 5{,}73 \text{ mm}$.
Für 20 % der Ringe: $1{,}02 \cdot 5{,}73 \text{ mm} < L_S^i \leq 1{,}10 \cdot 5{,}73 \text{ mm}$.

Die Selektivität der resultierenden Wertproduktbildung aus Acrolein und Acrylsäure betrug unter ansonsten identischen Betriebsbedingungen 96,2 mol-%.

**Patentansprüche**

**1.** Verfahren zur Beschickung eines Längsabschnitts eines Kontaktrohres mit einem einheitlichen Katalysatorfestbettabschnitt, dessen Aktivmasse wenigstens ein Multielementoxid ist, das

a) die Elemente Mo, Fe und Bi, oder
b) die Elemente Mo und V, oder
c) das Element V sowie zusätzlich P und/oder Sb

enthält, oder dessen Aktivmasse elementares Silber auf einem oxidischen Trägerkörper enthält, und der aus einer einzigen Sorte $S^i$ von ringförmigen Katalysatorformkörpern oder aus einem homogenisierten Gemisch voneinander unterscheidbarer Sorten $S^i$ von nur einer Sorte ringförmiger Katalysatorformkörper und nur einer Sorte ringförmiger Inertformkörper besteht, wobei der Median der Längstausdehnungen $L_S^i$ der geometrischen Formkörper einer Sorte $S^i$ einen Wert $D_S^i$ aufweist, **dadurch gekennzeichnet, dass** innerhalb der jeweiligen Sorte $S^i$ von geometrischen Formkörpern die Maßgabe M, dass

| | |
|---|---|
| 40 bis 70 % | der Gesamtzahl der zu $S^i$ gehörigen geometrischen Formkörper eine Längstausdehnung $L_S^i$ aufweisen, für die gilt $0{,}98 \cdot D_S^i \leq L_S^i \leq 1{,}02 \cdot D_S^i$, |
| wenigstens 10% | der Gesamtzahl der zu $S^i$ gehörigen geometrischen Formkörper eine Längstausdehnung $L_S^i$ aufweisen, für die gilt $0{,}94 \cdot D_S^i \leq L_S^i < 0{,}98 \cdot D_S^i$, |
| wenigstens 10 % | der Gesamtzahl der zu $S^i$ gehörigen geometrischen Formkörper eine Längstausdehnung $L_S^i$ aufweisen, für die gilt $1{,}02 \cdot D_S^i < L_S^i \leq 1{,}10 \cdot D_S^i$, |
| weniger als 5 % | der Gesamtzahl der zu $S^i$ gehörigen geometrischen Formkörper eine Längstausdehnung $L_S^i$ aufweisen, für die gilt $0{,}94 \cdot D_S^i > L_S^i$, und |
| weniger als 5 % | der Gesamtzahl der zu $S^i$ gehörigen geometrischen Formkörper eine Längstausdehnung $L_S^i$ aufweisen, für die gilt $1{,}10 \cdot D_S^i < L_S^i$, |

erfüllt ist.

**2.** Verfahren nach Anspruch 1, **dadurch gekennzeichnet, dass** innerhalb der jeweiligen Sorte $S^i$ von geometrischen Formkörpern die Maßgabe M*, dass

| | |
|---|---|
| 50 bis 60 % | der Gesamtzahl der zu $S^i$ gehörigen geometrischen Formkörper eine Längstausdehnung $L_S^i$ aufweisen, für die gilt $0{,}98 \cdot D_S^i \leq L_S^i \leq 1{,}02 \cdot D_S^i$, |
| wenigsten 15 % | der Gesamtzahl der zu $S^i$ gehörigen geometrischen Formkörper eine Längstausdehnung $L_S^i$ aufweisen, für die gilt $0{,}94 \cdot D_S^i \leq L_S^i < 0{,}98 \cdot D_S^i$, |
| wenigstens 15 % | der Gesamtzahl der zu $S^i$ gehörigen geometrischen Formkörper eine Längstausdehnung $L_S^i$ aufweisen, für die gilt $1{,}02 \cdot D_S^i < L_S^i \leq 1{,}10 \cdot D_S^i$, |
| weniger als 5 % | der Gesamtzahl der zu $S^i$ gehörigen geometrischen Formkörper eine Längstausdehnung $L_S^i$ aufweisen, für die gilt $0{,}94 \cdot D_S^i > L_S^i$, und |
| weniger als 5 % | der Gesamtzahl der zu $S^i$ gehörigen geometrischen Formkörper eine Längstausdehnung $L_S^i$ aufweisen, für die gilt $1{,}10 \cdot D_S^i < L_S^i$, |

erfüllt ist.

**3.** Verfahren nach Anspruch 1, **dadurch gekennzeichnet, dass** innerhalb der jeweiligen Sorte $S^i$ von geometrischen Formkörpern die Maßgabe M**, dass

| | |
|---|---|
| 50 bis 60 % | der Gesamtzahl der zu $S^i$ gehörigen geometrischen Formkörper eine Längstausdehnung $L_S^i$ aufweisen, für die gilt $0{,}98 \cdot D_S^i \leq L_S^i \leq 1{,}02 \cdot D_S^i$, |
| wenigsten 20 % | der Gesamtzahl der zu $S^i$ gehörigen geometrischen Formkörper eine Längstausdehnung $L_S^i$ aufweisen, für die gilt $0{,}94 \cdot D_S^i \leq L_S^i < 0{,}98 \cdot D_S^i$, |
| wenigstens 20 % | der Gesamtzahl der zu $S^i$ gehörigen geometrischen Formkörper eine Längstausdehnung $L_S^i$ aufweisen, für die gilt $1{,}02 \cdot D_S^i < L_S^i \leq 1{,}10 \cdot D_S^i$, |
| weniger als 5 % | der Gesamtzahl der zu $S^i$ gehörigen geometrischen Formkörper eine Längstausdehnung $L_S^i$ aufweisen, für die gilt $0{,}94 \cdot D_S^i > L_S^i$, und |
| weniger als 5 % | der Gesamtzahl der zu $S^i$ gehörigen geometrischen Formkörper eine Längstausdehnung $L_S^i$ aufweisen, für die gilt $1{,}10 \cdot D_S^i < L_S^i$, |

erfüllt ist.

**4.** Verfahren zur Beschickung eines Kontaktrohres mit einem Katalysatorfestbett, das aus mehreren aufeinanderfolgenden und voneinander verschiedenen in sich jeweils einheitlichen katalytisch aktiven Katalysatorfestbettabschnitten besteht und wobei die Aktivmasse aller Katalysatorfestbettabschnitte wenigstens ein Multielementoxid ist, das

    a) die Elemente Mo, Fe und Bi, oder
    b) die Elemente Mo und V, oder
    c) das Element V sowie zusätzlich P und/oder Sb

enthält, oder dessen Aktivmasse elementares Silber auf einem oxidischen Trägerkörper enthält, und der einzelne Katalysatorfestbettabschnitt aus einer einzigen Sorte $S^i$ von ringförmigen Katalysatorformkörpern oder aus einem homogenisierten Gemisch voneinander unterscheidbarer Sorten $S^i$ von nur einer Sorte ringförmiger Katalysatorformkörper und nur einer Sorte ringförmiger Inertformkörper besteht, **dadurch gekennzeichnet, dass** in jedem einzelnen Katalysatorfestbettabschnitt alle Sorten $S^i$ der in ihm jeweils enthaltenen geometrischen Formkörper die Maßgabe M gemäß Anspruch 1 erfüllen, alle geometrischen Formkörper die gleiche Ringgeometrie aufweisen und der gemeinsame Median $D^{Inert}_{Kat}$, gebildet über die Gesamtzahl G aus allen Längstausdehnungen $L_{Kat}$ der geometrischen Katalysatorformkörper und allen Längstausdehnungen $L_{Inert}$ der geometrischen Inertformkörper, und die Längstausdehnungen $L_{Inert}$ und $L_{Kat}$ (d. h., $L_{Kat,Inert}$) die Maßgabe $M^{G*}$, dass

| 40 bis 70 % | der Gesamtzahl G eine Längstausdehnung $L_{Kat,Inert}$ aufweisen, für die gilt $0{,}98 \cdot D^{Inert}_{Kat} \le L_{Kat,Inert} \le 1{,}02 \cdot D^{Inert}_{Kat}$, |
|---|---|
| wenigstens 10 % | der Gesamtzahl G eine Längstausdehnung $L_{Kat,Inert}$ aufweisen, für die gilt $0{,}94 \cdot D^{Inert}_{Kat} \le L_{Kat,Inert} < 0{,}98 \cdot D^{Inert}_{Kat}$, |
| wenigstens 10 % | der Gesamtzahl G eine Längstausdehnung $L_{Kat,Inert}$ aufweisen, für die gilt $1{,}02 \cdot D^{Inert}_{Kat} < L_{Kat,Inert} \le 1{,}10 \cdot D^{Inert}_{Kat}$, |
| weniger als 5 % | der Gesamtzahl G eine Längstausdehnung $L_{Kat,Inert}$ aufweisen, für die gilt $0{,}94 \cdot D^{Inert}_{Kat} > L_{Kat,Inert}$, und |
| weniger als 5 % | der Gesamtzahl G eine Längstausdehnung $L_{Kat,Inert}$ aufweisen, für die gilt $1{,}10 \cdot D^{Inert}_{Kat} < L_{Kat,Inert}$, |

erfüllen.

5. Rohrbündelreaktor enthaltend wenigstens ein Kontaktrohr, das nach einem Verfahren gemäß einem der Ansprüche 1 bis 4 beschickt worden ist.

6. Verfahren der heterogen katalysierten partiellen Gasphasenoxidation einer organischen Verbindung in einem Rohrbündelreaktor, **dadurch gekennzeichnet, dass** der Rohrbündelreaktor ein Rohrbündelreaktor gemäß Anspruch 5 ist.

7. Verfahren nach Anspruch 6, **dadurch gekennzeichnet, dass** die heterogen katalysierte partielle Gasphasenoxidation diejenige von Propylen zu Acrolein und/oder diejenige von Acrolein zu Acrylsäure ist.

**Claims**

1. A process for charging a longitudinal section of a catalyst tube with a uniform fixed catalyst bed section whose active composition is at least one multielement oxide which comprises

  a) the elements Mo, Fe and Bi, or
  b) the elements Mo and V, or
  c) the element V and additionally P and/or Sb,

or whose active composition comprises elemental silver on an oxidic support body, and which consists of a single type $S^i$ of annular shaped catalyst bodies or of a homogenized mixture of mutually distinguishable types $S^i$ of only one type of annular shaped catalyst bodies and only one type of annular shaped inert bodies, where the median of the longest dimensions $L_S^i$ of the geometric shaped bodies of one type $S^i$ has a value $D_S^i$, wherein, within the respective type $S^i$ of geometric shaped bodies, the proviso M that
from 40 to 70% of the total number of the geometric shaped bodies belonging to $S^i$ has a longest dimension $L_S^i$ for which

$$0.98 \cdot D_S^i \le L_S^i \le 1.02 \cdot D_S^i,$$

at least 10% of the total number of the geometric shaped bodies belonging to $S^i$ has a longest dimension $L_S^i$ for which

$$0.94 \cdot D_S^i \le L_S^i < 0.98 \cdot D_S^i,$$

at least 10% of the total number of the geometric shaped bodies belonging to $S^i$ has a longest dimension $L_S^i$ for which

$$1.02 \cdot D_S^i < L_S^i \leq 1.10 \cdot D_S^i,$$

less than 5% of the total number of the geometric shaped bodies belonging to $S^i$ has a longest dimension $L_S^i$ for which

$$0.94 \cdot D_S^i > L_S^i,$$

and
less than 5% of the total number of the geometric shaped bodies belonging to $S^i$ has a longest dimension $L_S^i$ for which

$$1.10 \cdot D_S^i < L_S^i$$

is satisfied.

2. The process according to claim 1, wherein, within the respective type $S^i$ of geometric shaped bodies, the proviso M* that
from 50 to 60% of the total number of the geometric shaped bodies belonging to $S^i$ has a longest dimension $L_S^i$ for which

$$0.98 \cdot D_S^i \leq L_S^i \leq 1.02 \cdot D_S^i,$$

at least 15% of the total number of the geometric shaped bodies belonging to $S^i$ has a longest dimension $L_S^i$ for which

$$0.94 \cdot D_S^i \leq L_S^i < 0.98 \cdot D_S^i,$$

at least 15% of the total number of the geometric shaped bodies belonging to $S^i$ has a longest dimension $L_S^i$ for which

$$1.02 \cdot D_S^i < L_S^i \leq 1.10 \cdot D_S^i,$$

less than 5% of the total number of the geometric shaped bodies belonging to $S^i$ has a longest dimension $L_S^i$ for which

$$0.94 \cdot D_S^i > L_S^i,$$

and
less than 5% of the total number of the geometric shaped bodies belonging to $S^i$ has a longest dimension $L_S^i$ for which

$$1.10 \cdot D_S^i < L_S^i$$

is satisfied.

3. The process according to claim 1, wherein, within the respective type $S^i$ of geometric shaped bodies, the proviso M** that
from 50 to 60% of the total number of the geometric shaped bodies belonging to $S^i$ has a longest dimension $L_S^i$ for which

$$0.98 \cdot D_S^i \leq L_S^i \leq 1.02 \cdot D_S^i,$$

at least 20% of the total number of the geometric shaped bodies belonging to $S^i$ has a longest dimension $L_S^i$ for which

$$0.94 \bullet D_S^i \leq L_S^i < 0.98 \bullet D_S^i,$$

at least 20% of the total number of the geometric shaped bodies belonging to $S^i$ has a longest dimension $L_S^i$ for which

$$1.02 \bullet D_S^i < L_S^i \leq 1.10 \bullet D_S^i,$$

less than 5% of the total number of the geometric shaped bodies belonging to $S^i$ has a longest dimension $L_S^i$ for which

$$0.94 \bullet D_S^i > L_S^i,$$

and

less than 5% of the total number of the geometric shaped bodies belonging to $S^i$ has a longest dimension $L_S^i$ for which

$$1.10 \bullet D_S^i < L_S^i$$

is satisfied.

4. A process for charging a catalyst tube with a fixed catalyst bed which consists of a plurality of successive and mutually different catalytically active fixed catalyst bed sections, each of which is intrinsically homogeneous, and where the active composition of all fixed catalyst bed sections comprises at least one multielement oxide which comprises

    a) the elements Mo, Fe and Bi, or
    b) the elements Mo and V, or
    c) the element V and additionally P and/or Sb,

or whose active composition comprises elemental silver on an oxidic support body, and the individual fixed catalyst bed section consists of a single type $S^i$ of annular shaped catalyst bodies or of a homogenized mixture of mutually distinguishable types $S^i$ of only one type of annular shaped catalyst bodies and only one type of annular shaped inert bodies, wherein, in each individual fixed catalyst bed section, all types $S^i$ of geometric shaped bodies present therein in each case satisfy the proviso M according to claim 1, all geometric shaped bodies have the same ring geometry and the combined medium $D^{inert}_{cat}$, formed over the total number G of all longest dimensions $L_{cat}$ of the geometric shaped catalyst bodies and all longest dimensions $L_{inert}$ of the geometric shaped inert bodies, and the longest dimensions $L_{inert}$ and $L_{cat}$ (i.e. $L_{cat,inert}$) satisfy the proviso $M^{G*}$ that
from 40 to 70% of the total number G has a longest dimension $L_{cat,inert}$ for which

$$0.98 \bullet D^{inert}_{cat} \leq L_{cat,inert} \leq 1.02 \bullet D^{inert}_{cat},$$

at least 10% of the total number G has a longest dimension $L_{cat,inert}$ for which

$$0.94 \bullet D^{inert}_{cat} \leq L_{cat,inert} < 0.98 \bullet D^{inert}_{cat},$$

at least 10% of the total number G has a longest dimension $L_{cat,inert}$ for which

$$1.02 \bullet D^{inert}_{cat} < L_{cat,inert} \leq 1.10 \bullet D^{inert}_{cat},$$

less than 5% of the total number G has a longest dimension $L_{cat,inert}$ for which

$$0.94 \cdot D^{inert}_{cat} > L_{cat,inert},$$

and
less than 5% of the total number G has a longest dimension $L_{cat,inert}$ for which

$$1.10 \cdot D^{inert}_{cat} < L_{cat,inert}.$$

5. A tube bundle reactor comprising at least one catalyst tube which has been charged by a process according to any of claims 1 to 4.

6. A process for heterogeneously catalyzed partial gas phase oxidation of an organic compound in a tube bundle reactor, wherein the tube bundle reactor is a tube bundle reactor according to claim 5.

7. The process according to claim 6, wherein the heterogeneously catalyzed partial gas phase oxidation is that of propylene to acrolein and/or that of acrolein to acrylic acid.


**Revendications**

1. Procédé de chargement d'une section longitudinale d'un tube de contact avec une section de lit catalytique fixe uniforme, dont la masse active est au moins un oxyde de plusieurs éléments, qui contient :

   a) les éléments Mo, Fe et Bi, ou
   b) les éléments Mo et V, ou
   c) l'élément V, ainsi qu'en outre P et/ou Sb,

   ou dont la masse active contient de l'argent élémentaire sur un corps support oxydique, et qui est constituée par un type unique $S^i$ de corps moulés catalytiques annulaires ou par un mélange homogénéisé de types $S^i$ distincts les uns des autres d'uniquement un type de corps moulés catalytiques annulaires et d'uniquement un type de corps moulés inertes annulaires, la médiane des dimensions longitudinales $L_S^i$ des corps moulés géométriques d'un type $S^i$ présentant une valeur $D_S^i$, **caractérisé en ce qu'**au sein de chaque type $S^i$ de corps moulés géométriques, la condition M selon laquelle
   40 à 70 % du nombre total des corps moulés géométriques appartenant à $S^i$ présentent une dimension longitudinale $L_S^i$, pour laquelle : $0,98 \cdot D_S^i \leq L_S^i \leq 1,02 \cdot D_S^i$,
   au moins 10 % du nombre total des corps moulés géométriques appartenant à $S^i$ présentent une dimension longitudinale $L_S^i$, pour laquelle : $0,94 \cdot D_S^i \leq L_S^i < 0,98 \cdot D_S^i$,
   au moins 10 % du nombre total des corps moulés géométriques appartenant à $S^i$ présentent une dimension longitudinale $L_S^i$, pour laquelle : $1,02 \cdot D_S^i < L_S^i \leq 1,10 \cdot D_S^i$,
   moins de 5 % du nombre total des corps moulés géométriques appartenant à $S^i$ présentent une dimension longitudinale $L_S^i$, pour laquelle : $0,94 \cdot D_S^i > L_S^i$, et
   moins de 5 % du nombre total des corps moulés géométriques appartenant à $S^i$ présentent une dimension longitudinale $L_S^i$, pour laquelle : $1,10 \cdot D_S^i < L_S^i$,
   est remplie.

2. Procédé selon la revendication 1, **caractérisé en ce qu'**au sein de chaque type $S^i$ de corps moulés géométriques, la condition M* selon laquelle
   50 à 60 % du nombre total des corps moulés géométriques appartenant à $S^i$ présentent une dimension longitudinale $L_S^i$, pour laquelle : $0,98 \cdot D_S^i \leq L_S^i \leq 1,02 \cdot D_S^i$,
   au moins 15 % du nombre total des corps moulés géométriques appartenant à $S^i$ présentent une dimension longitudinale $L_S^i$, pour laquelle : $0,94 \cdot D_S^i \leq L_S^i < 0,98 \cdot D_S^i$,
   au moins 15 % du nombre total des corps moulés géométriques appartenant à $S^i$ présentent une dimension longitudinale $L_S^i$, pour laquelle : $1,02 \cdot D_S^i < L_S^i \leq 1,10 \cdot D_S^i$,
   moins de 5 % du nombre total des corps moulés géométriques appartenant à $S^i$ présentent une dimension longitudinale $L_S^i$, pour laquelle : $0,94 \cdot D_S^i > L_S^i$, et
   moins de 5 % du nombre total des corps moulés géométriques appartenant à $S^i$ présentent une dimension longi-

tudinale $L_S^i$, pour laquelle : $1{,}10 \cdot D_S^i < L_S^i$,
est remplie.

3. Procédé selon la revendication 1, **caractérisé en ce qu'**au sein de chaque type $S^i$ de corps moulés géométriques, la condition M** selon laquelle

50 à 60 % du nombre total des corps moulés géométriques appartenant à $S^i$ présentent une dimension longitudinale $L_S^i$, pour laquelle : $0{,}98 \cdot D_S^i \leq L_S^i \leq 1{,}02 \cdot D_S^i$,

au moins 20 % du nombre total des corps moulés géométriques appartenant à $S^i$ présentent une dimension longitudinale $L_S^i$, pour laquelle : $0{,}94 \cdot D_S^i \leq L_S^i < 0{,}98 \cdot D_S^i$,

au moins 20 % du nombre total des corps moulés géométriques appartenant à $S^i$ présentent une dimension longitudinale $L_S^i$, pour laquelle : $1{,}02 \cdot D_S^i < L_S^i \leq 1{,}10 \cdot D_S^i$,

moins de 5 % du nombre total des corps moulés géométriques appartenant à $S^i$ présentent une dimension longitudinale $L_S^i$, pour laquelle : $0{,}94 \cdot D_S^i > L_S^i$, et

moins de 5 % du nombre total des corps moulés géométriques appartenant à $S^i$ présentent une dimension longitudinale $L_S^i$, pour laquelle : $1{,}10 \cdot D_S^i < L_S^i$,
est remplie.

4. Procédé de chargement d'un tube de contact avec un lit catalytique fixe, qui est constitué par plusieurs sections de lit catalytique fixe catalytiquement actives successives et différentes les unes des autres, chacune uniformes en elles-mêmes, la masse active de toutes les sections de lit catalytique fixe étant au moins un oxyde de plusieurs éléments, qui contient :

   a) les éléments Mo, Fe et Bi, ou
   b) les éléments Mo et V, ou
   c) l'élément V, ainsi qu'en outre P et/ou Sb,

   ou la masse active contenant de l'argent élémentaire sur un corps support oxydique, et la section de lit catalytique fixe individuelle étant constituée par un type unique $S^i$ de corps moulés catalytiques annulaires ou par un mélange homogénéisé de types $S^i$ distincts les uns des autres d'uniquement un type de corps moulés catalytiques annulaires et d'uniquement un type de corps moulés inertes annulaires, **caractérisé en ce que**, dans chaque section de lit catalytique fixe individuelle, tous les types $S^i$ des corps moulés géométriques contenus dans celle-ci remplissent la condition M selon la revendication 1, tous les corps moulés géométriques présentent la même géométrie annulaire, et la médiane commune $D^{inerte}_{cat}$, formée par le nombre total G de toutes les dimensions longitudinales $L_{cat}$ des corps moulés catalytiques géométriques et de toutes les dimensions longitudinales $L_{inerte}$ des corps moulés inertes géométriques, et les dimensions longitudinales Linerte et $L_{cat}$ (c.-à-d. $L_{cat, inerte}$) remplissent la condition $M^{G*}$ selon laquelle

   40 à 70 % du nombre total G présentent une dimension longitudinale $L_{cat,inerte}$, pour laquelle : $0{,}98 \cdot D^{inerte}_{cat} \leq L_{cat,inerte} \leq 1{,}02 \cdot D^{inerte}_{cat}$,

   au moins 10 % du nombre total G présentent une dimension longitudinale $L_{cat,inerte}$, pour laquelle : $0{,}94 \cdot D^{inerte}_{cat} \leq L_{cat,inerte} < 0{,}98 \cdot D^{inerte}_{cat}$,

   au moins 10 % du nombre total G présentent une dimension longitudinale $L_{cat,inerte}$, pour laquelle : $1{,}02 \cdot D^{inerte}_{cat} < L_{cat,inerte} \leq 1{,}10 \cdot D^{inerte}_{cat}$,

   moins de 5 % du nombre total G présentent une dimension longitudinale $L_{cat,inerte}$, pour laquelle : $0{,}94 \cdot D^{inerte}_{cat} > L_{cat,inerte}$, et

   moins de 5 % du nombre total G présentent une dimension longitudinale $L_{cat,inerte}$, pour laquelle : $1{,}10 \cdot D^{inerte}_{cat} < L_{cat,inerte}$.

5. Réacteur à faisceau de tubes contenant au moins un tube de contact, qui a été chargé par un procédé selon l'une quelconque des revendications 1 à 4.

6. Procédé d'oxydation partielle en phase gazeuse sous catalyse hétérogène d'un composé organique dans un réacteur à faisceau de tubes, **caractérisé en ce que** le réacteur à faisceau de tubes est un réacteur à faisceau de tubes selon la revendication 5.

7. Procédé selon la revendication 6, **caractérisé en ce que** l'oxydation partielle en phase gazeuse sous catalyse hétérogène est celle de propylène en acroléine et/ou celle d'acroléine en acide acrylique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2004225138 A1
- EP 1270065 A
- GB 1550036 A
- US 2004260103 A1
- DE 4431949 A
- EP 700714 A
- DE PS2830765 C
- DE 2201528 A
- DE 2231557 A
- DE 2310517 A
- DE 2830765 C
- DE 2513405 C
- US 3147084 A
- EP 383224 A
- DE 2903582 A
- EP 468290 A
- WO 03059857 A
- WO 03057653 A
- EP 873783 A
- DE 2351151 A
- DE 2526238 A
- EP 92097 A
- EP 58927 A
- DE 4132263 A
- DE 4132684 A
- DE 4022212 A
- EP 522871 A
- DE 2106796 A
- DE 1624921 A
- GB 1464198 A
- GB 1291354 A
- DE 2025430 A
- DE AS1254137 B
- DE 2159346 A
- EP 372972 A
- WO 890710 A
- DE 4311608 A
- DE 10131297 A
- EP 1090684 A
- EP 608838 A
- DE 10046672 A
- EP 529853 A
- WO 0196270 A
- DE 10028582 A
- US 20050263926 A
- US 20060205978 A
- US 20060161019 A
- EP 979813 A
- EP 090744 A
- EP 456837 A
- EP 1106598 A
- US 5198581 A
- US 4203903 A
- WO 2005113123 A
- EP 714700 A
- WO 0230569 A
- WO 2005030393 A
- WO 2005497012 A
- DE 102007005602 A
- DE 102007004961 A
- WO 200503093 A
- DE 102004025445 A
- EP 467144 A
- WO 03078310 A
- WO 0168245 A
- DE 102005035978 A
- DE 10350822 A
- DE 102007010422 A
- US 6528683 A
- US 6586361 A
- US 6362345 A
- EP 496470 A
- WO 2006094766 A
- JP 2004195279 A
- EP 1254707 A
- EP 1254710 A
- EP 1254709 A
- WO 2004035528 A
- DE 10248584 A
- DE 10254278 A
- DE 10254279 A
- WO 0206199 A
- WO 02051539 A